# EUROPEAN PATENT APPLICATION

(11) **EP 4 804 730 A1**
(43) Date of publication of application: **09.09.2026**
(21) Application number: 24884743.6
(22) Date of filing: 29.10.2024
(51) Int. Cl.: H05B 45/20

(54) **ILLUMINATION ADJUSTING SYSTEM AND METHOD FOR INFANT**

(30) Priority: 02.11.2023 US 202363595545 P
(71) Applicant: Lin, Chi-liang, Taoyuan, Taiwan 33045 (TW)
(72) Inventor: Lin, Chi-liang, Taoyuan, Taiwan 33045 (TW)
(74) Representative: Lang, Christian
(86) International application number: PCT/CN2024/128185
(87) International publication number: WO 2025/092740

(57) **Abstract**

This invention provides a lighting adjustment system for infants and toddlers. The system includes an adjustable light source that can control various lighting conditions such as spectral color temperature, brightness, flicker rate, and duration. Additionally, the system features a feedback mechanism to monitor the establishment of infants' sleep rhythms and cognitive responses. Furthermore, the invention includes a judgment software that automatically adjusts lighting conditions based on feedback data to improve the development curve of REM sleep, establish good and stable sleep rhythms, and thereby promote the cognitive development of child.

## Description

### FIELD OF THE INVENTION

This invention belongs to the field of infant and toddler care and lighting technology. Specifically, it provides a lighting adjustment system with a rhythmic lighting spectral formula. By illuminating infants and toddlers, it influences their sleep cycles and adjusts the spectral formula of the light based on changes in the rapid eye movement (REM) phase of their sleep cycles. This aims to enhance the sleep cycles of infants and toddlers and promote their future cognitive development.

### BACKGROUND OF THE INVENTION

In recent years, the relationship between sleep quality and cognitive development has received widespread attention in the field of research on the health and development of infants and toddlers. Most studies believe that stable and high-quality sleep can have a positive impact on the brain development and emotional stability of infants and toddlers. However, a review of current crib-related products reveals that existing lighting solutions for infants and toddlers are usually just simple light sources, often with only on/off and basic brightness adjustment functions. These simple lighting solutions are insufficient to provide a comprehensive and suitable light environment for infants and toddlers, which is necessary to promote their physical and mental health development.

In addition, some high-end smart lighting products have started to appear, but these products usually focus on lighting for adults or specific occasions and are not specifically designed for infants and toddlers. Even if there are products specifically for infants and toddlers, they are mostly limited to basic color temperature and brightness adjustments, without truly considering the unique physiological and psychological needs of infants and toddlers. Moreover, past research and products have lacked a deep understanding and practical application of the biological rhythms of infants and toddlers, especially the critical role of intrinsically photosensitive retinal ganglion cells (ipRGCs) in their development, which has been almost neglected in traditional lighting solutions. Clearly, existing or traditional crib lighting systems are mostly single-mode lighting, lacking sufficient flexibility and adaptability to meet the brain development and emotional stability needs of infants and toddlers.

Regarding the definition of sleep quality, according to AK Patel's 2022 work (Physiology, Sleep Stages), sleep is divided into five stages: wakefulness, N1, N2, N3, and REM. N1 to N3 are non-rapid eye movement (NREM) sleep stages, gradually leading to deeper sleep states. About 75% of sleep is NREM, especially the N2 stage. There are 4 to 5 sleep cycles each night, each lasting about 90 to 110 minutes. The REM stage is characterized by high-frequency, low-amplitude beta waves and is associated with dreaming. For example, REM can be easily observed through EEG (electroencephalogram) and used to define sleep quality.

Additionally, AK Patel also pointed out that in the first few weeks of life, the sleep time of newborns is randomly distributed between day and night, accompanied by irregular sleep and wake patterns. Newborns sleep about 16 to 18 hours a day, but this is intermittent, with the longest continuous sleep period usually being 2.5 to 4 hours. Newborns have three different types of sleep: quiet sleep (similar to NREM), active sleep (similar to REM), and indeterminate sleep. More specifically, the sleep of newborns differs from that of children and adults; newborns start their sleep with the REM stage rather than the NREM stage, and each sleep cycle includes only 1 to 2 cycles. These differences in sleep and sleep stages occur because the circadian rhythms of newborns or infants and toddlers have not yet formed.

Therefore, there is an urgent need to develop a lighting adjustment system that can more effectively promote healthy sleep and cognitive development in infants and toddlers. This system not only needs to provide a diverse and adjustable rhythmic spectral formula but also must be able to self-regulate based on the physiological and behavioral feedback of infants and toddlers to achieve optimal results.

### SUMMARY OF THE INVENTION

Since newborns or infants and toddlers have not yet formed circadian rhythms, their sleep time is randomly distributed between day and night, accompanied by irregular sleep and wake patterns. Therefore, this invention primarily provides a lighting adjustment system with a rhythmic lighting spectral formula. By illuminating infants and toddlers, it influences their sleep cycles and adjusts the spectral formula of the light based on changes in the rapid eye movement (REM) phase of their sleep cycles. This aims to enhance the sleep cycles of infants and toddlers and promote their future cognitive development.

According to the above description, one of the main objectives of this invention is to provide a method for illuminating infants and toddlers, which includes:
providing a lighting adjustment system, consists of a light source adjustment device, a feedback device, and a servo device, the servo device stores a rhythmic lighting formula related to perceptual development and a REM sleep development curve, used to drive the light source adjustment device to provide lighting according to the rhythmic lighting formula, the feedback device comes into contact with the infant or toddler to transmit the number of REM sleep hours, after light therapy, to the servo device;
executing the illumination procedure, driven by the servo device, which adjusts the light source according to the rhythmic lighting formula to sequentially illuminate the infant; obtaining the number of REM sleep hours of the infant or toddler, transmitted by the feedback device to the servo device after illumination;
evaluating whether the REM sleep state meets expectations, by comparing the number of REM sleep hours transmitted by the feedback device with the REM sleep development curve stored in the servo device, and
maintaining continuous illumination according to the rhythmic lighting formula, the servo device determines that the number of REM sleep hours of the infant or toddler meets the REM sleep development curve, it drives the light source adjustment device to continue illuminating the infant or toddler according to the rhythmic lighting formula.

Furthermore, another main objective of this invention is to provide a lighting adjustment system for infants and toddlers, which includes:
a crib device, used to provide a sleeping space for the infant;
a light source adjustment device, equipped with LED lights, positioned above or around the crib device, used to adjust the lighting parameters of the LED lights;
a feedback device, placed on the body of the infant or toddler, used to obtain and transmit the real-time REM sleep state of the infant or toddler; and
a servo device, equipped with a processing unit, a communication unit, and a memory unit. The memory unit stores a rhythmic lighting formula related to perceptual development and a REM sleep development curve. The processing unit is used to drive the light source adjustment device to illuminate the infant or toddler based on the comparison and evaluation of the real-time REM sleep state with the REM sleep development curve.

It is evident that this invention targets infants and toddlers whose intrinsically photosensitive retinal ganglion cells (ipRGCs) have not yet established cycles influenced by actual lighting conditions. By providing early periodic stimulation, it can help newborns establish a more resilient rhythmic cycle. This resilience allows for quicker correction of rhythm disturbances caused by staying up late or occasional jet lag as infants and toddlers grow. Additionally, it enhances the robustness of their nervous systems.

### BRIEF DESCRIPTION OF THE DRAWINGS

Figure 1 is a schematic diagram of the emotion induction mechanism.
Figures 2A-2D are schematic diagrams of the corresponding color temperature curves for brain regions according to the invention.
Figure 3A is a schematic diagram of the stimulation results on brain regions using pure red light.
Figure 3B is a schematic diagram of the stimulation results on brain regions using green-red light.
Figure 3C is a schematic diagram of the stimulation results on brain regions using blue-red light.
Figure 3D is a schematic diagram of the stimulation results on brain regions using white light with a color temperature of 3000K.
Figure 3E is a schematic diagram of the stimulation results on brain regions using white light with a color temperature of 4000K.
Figure 3F is a schematic diagram of the stimulation results on brain regions using white light with a color temperature of 5700K.
Figure 4 is a schematic diagram of the spectrum selected for illuminating infants and toddlers.
Figure 5 is a schematic diagram of the age and REM development curve.
Figure 6 is an infant lighting adjustment system according to the invention.
Figure 7 is a flowchart of the operation of the infant lighting adjustment system according to the invention.

### DETAILED DESCRIPTION OF THE PREFERRED EMBODIMENT

This invention uses fMRI to study the stimulation of different lighting conditions on the brain region of the subjects, discovering that lighting can affect emotions, visual development, cognitive responses, and sleep cycles. It can also be proven that merely adjusting the lighting conditions (i.e., illumination) can not only change a person's emotions, as commonly known, but also further enhance the visual development, cognitive responses, and sleep cycles of animals. Additionally, in this invention, the term 'infant' includes newborns (i.e., within 3 months after birth), infants (i.e., from 3 to 12 months after birth).

First, this invention conducted a set of lighting experiments purely using color temperature adjustment to observe brain regions, particularly a set of experiments without viewing emotional images, purely providing color temperature adjustment to observe brain regions. In the experiments conducted in Kaohsiung, Taiwan, and Beijing, China, we obtained a set of blood-oxygen-level-dependent (BOLD) indices for brain regions under 3000K, 4000K, and 5700K lighting conditions. The BOLD indices for brain regions under 3000K, 4000K, and 5700K lighting conditions are shown in Table 1.

**Table 1**

| Brain Region | 3000K | 4000K | 5700K |
|---|---|---|---|
| Calcarine | 113 | 0 | 296 |
| Frontal Superior | -206 | 236 | -45 |
| IFG | 40 | 365 | -49 |
| MCC | -83 | 22 | -91 |

Among them, the left calcarine cortex represents negative emotions; the left superior frontal gyrus represents emotional arousal (neuroticism); the left middle cingulate cortex (MCC) and the right inferior frontal gyrus (IFG) represent stability. Next, Table 1 is converted into the blood-oxygen-level-dependent (BOLD) signal indices relative to color temperature and emotions, as shown in Table 2. In Table 2, the emotional stability index is obtained by adding the MCC (left middle cingulate cortex) and the IFG (right inferior frontal gyrus).

**Table 2**

| Emotion | 3000K | 4000K | 5700K |
|---|---|---|---|
| Negative emotions | 113 | 0 | 296 |
| Emotional arousal | -206 | 236 | -45 |
| Emotional stability | -43 | 387 | -140 |

Since emotional arousal and emotional stability are opposing emotions, we subtract one from the other as an opposing emotional indicator; the opposite of negative emotion is positive emotion. Therefore, we can summarize the BOLD indices for emotions under 3000K, 4000K, and 5700K color temperatures as shown in Table 3.

**Table 3**

| Emotion | 3000K | 4000K | 5700K |
|---|---|---|---|
| Degree of positive emotion | -113 | 0 | -296 |
| Degree of emotional stability | 163(=-43+206) | 151(=387-236) | -95(=-140+45) |

Based on the above results, the following conclusions can be drawn:
1. 4000K, compared to other color temperatures, can give people more positive emotions, while 5700K is most likely to bring negative emotions.
2. 3000K gives a sense of stability, and 5700K, compared to other color temperatures, is more likely to cause emotional arousal.

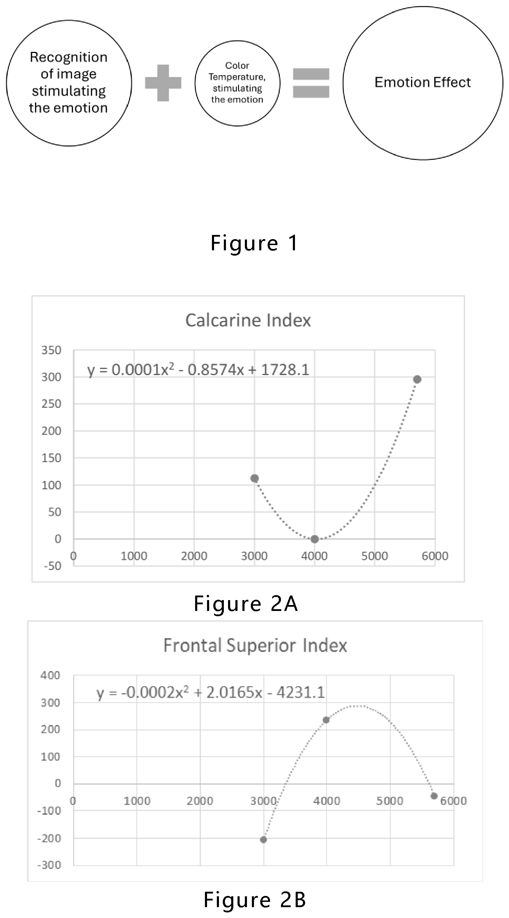

It is evident from the above experimental results that adjusting the color temperature of pure light can also affect emotions. Furthermore, based on the above, this invention considers that a complete emotional induction system includes the impact of 'image cognition' on emotions and the impact of 'color temperature change' on emotions. According to the above experimental results, it can be found that using pure light illumination and using emotional images for emotional guidance, both 'image cognition' and 'color temperature change' can affect emotions. However, the impact of 'color temperature change' on emotions is not as significant as that of emotional images. This invention confirms that the impact of emotional induction includes 'image cognition' and 'color temperature change,' with 'image cognition' having a greater impact on emotions than 'color temperature change,' as shown in Figure 1, which is a schematic diagram of the emotional induction mechanism.

It should be noted that, based on the above, this invention determines that changes in color temperature can only serve as an auxiliary adjustment for emotions, while the primary emotional changes stem from cognitive stimuli. In this invention, emotional stimulation is induced using an emotional image database (International Affective Picture System, IAPS). Furthermore, following the pure color experiments conducted as part of this invention, we identified the corresponding color temperature curves for four specific brain regions, which are described as follows.

First, regarding the experiment on the left calcarine cortex brain region. According to Table 1, the BOLD indices of the left calcarine cortex brain region under different color temperature illuminations are shown in Table 4.

**Table 4**

| Color temperature(K) | 3000 | 4000 | 5700 |
|---|---|---|---|
| Calcarine Index | 113 | 0 | 296 |

This invention derived the corresponding color temperature curve for the calcarine cortex (representing negative emotions) brain region, as shown in Figure 2A. It is evident that the calcarine cortex corresponding color temperature curve has a minimum value at 4000K; conversely, when the color temperature increases or decreases, it causes more negative emotions.

Next, regarding the experiment on the left superior frontal gyrus (Frontal Superior) brain region. According to Table 1, the BOLD indices of the Frontal Superior under different color temperature illuminations are shown in Table 5.

**Table 5**

| Color temperature(K) | 3000 | 4000 | 5700 |
|---|---|---|---|
| Frontal Superior Index | -206 | 236 | -45 |

This invention derived the corresponding color temperature curve for the Frontal Superior (representing emotional arousal) brain region, as shown in Figure 2B. It is evident that the Frontal Superior has a maximum value at 4400K, which can elevate emotions. Next, regarding the experiment on the right inferior frontal gyrus (IFG) brain region. According to Table 1, the BOLD indices of the IFG under different color temperature illuminations are shown in Table 6.

**Table 6**

| Color temperature(K) | 3000 | 4000 | 5700 |
|---|---|---|---|
| IFG Index | 40 | 365 | -49 |

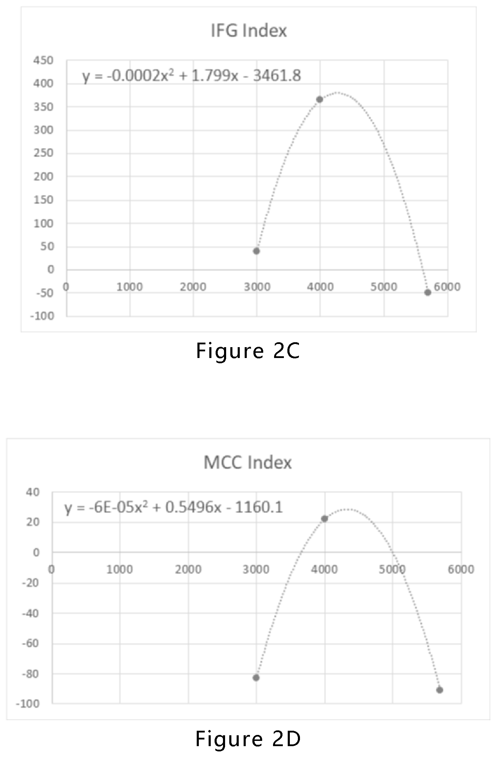

This invention derived the corresponding color temperature curve for the IFG (representing emotional stability) brain region, as shown in Figure 2C. It is evident that the IFG (representing emotional stability) has a maximum value at 4200K. Next, regarding the experiment on the MCC (left middle cingulate cortex) brain region. According to Table 1, the BOLD indices of the MCC under different color temperature illuminations are shown in Table 7.

**Table 7**

| Color temperature(K) | 3000 | 4000 | 5700 |
|---|---|---|---|
| MCC | -83 | 22 | -91 |

We derived the corresponding color temperature curve for the MCC brain region, as shown in Figure 2D. It is evident that the MCC, like the IFG, represents stability and also has a maximum value at 4200K.

The functions of brain regions and critical conditions are summarized in Table 8. It was found that adjusting the color temperature affects the following four brain regions: the left calcarine cortex (Calcarine), which represents negative emotions and, more importantly, visual responses in cognitive functions; the left superior frontal gyrus (Frontal Superior), which represents emotional arousal (neuroticism) and, more importantly, working memory and laughter in cognitive functions; the left middle cingulate cortex (MCC) and the right inferior frontal gyrus (IFG), both representing emotional stability. In the left middle cingulate cortex (MCC), it is more important for cognition and reward in cognitive functions, while in the right inferior frontal gyrus (IFG), it is more important for language in cognitive functions.

**Table 8**

| Brain Region | Cognitive function | Emotional function | Color temperature threshold condition |
|---|---|---|---|
| Calcarine (left calcarine cortex) | Visual response | negative emotions | Min: 4000K |
| Frontal Superior (left superior frontal gyrus) | Working memory, Laughter | emotional arousal | Max: 4400K |
| IFG (right inferior frontal gyrus) | Language | stable | Max: 4200K |
| MCC (left middle cingulate cortex) | Cognition and reward | stable | Max: 4200K |

Next, this invention further investigates the effects of pure light stimulation on brain regions. The experiment involved 30 participants and tested green light, red light, blue light, and three white lights at 3000K, 4000K, and 5700K. The results are described as follows.

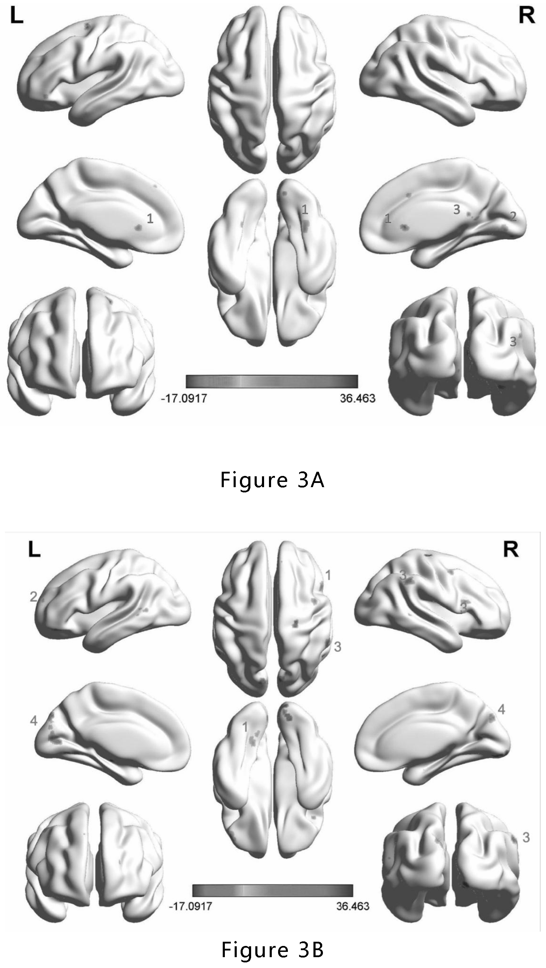

First, the stimulation results of the brain region after using pure red light illumination are shown in Figure 3A. In Figure 3A, the phototransduction signal (i.e., stimulation response) of the low-level visual brain region can be obtained in the right brain region, as shown at position 2 in Figure 3A. Additionally, the language-related brain region in the right brain region is stimulated, which can enhance expressive ability, as shown at position 3 in Figure 3A.

Next, the stimulation results of the brain region after using green light illumination are shown in Figure 3B. In Figure 3B, an increase in the blood flow response in the creativity-related area of the right brain region can be observed, as shown at position 2 in Figure 3B. Additionally, the language expression ability-related area in the right brain region is significantly stimulated, as shown at position 3 in Figure 3B. Furthermore, in the visual response area of the right brain region, the responses of both low-level and high-level visual transmission (including the meaning generated by images) are enhanced. The low-level visual response enhances expressive ability, while the high-level visual response enhances the ability to convert images into meaning, as shown at position 4 in Figure 3B.

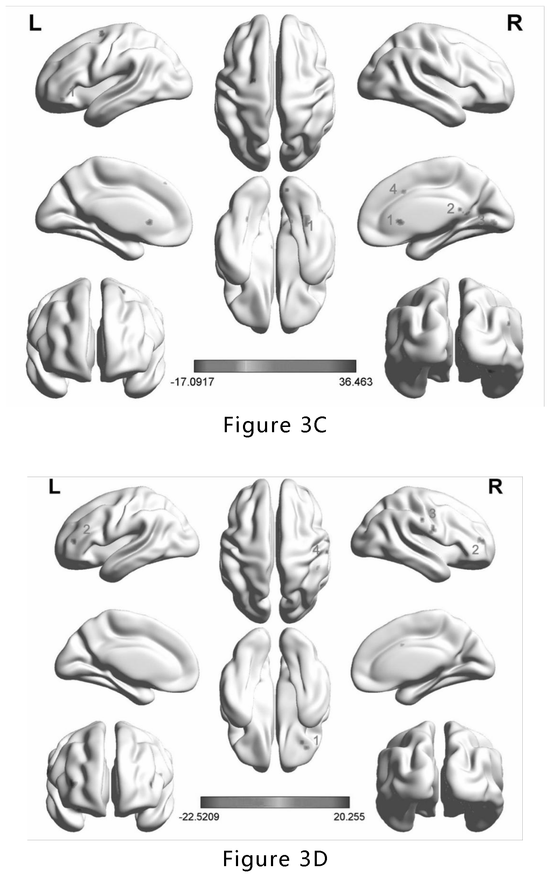

Then, the stimulation results of the brain region after using blue light illumination are shown in Figure 3C. In Figure 3C, a response in the language area of the right brain region can be observed, as shown at position 2 in Figure 3C. Additionally, the low-level visual response area in the right brain region is stimulated, enhancing expressive ability, as shown at position 3 in Figure 3C. Furthermore, an arousal response in the right brain region can be observed, as shown at position 4 in Figure 3C.

Subsequently, the stimulation results of the brain region after using white light with a color temperature of 3000K are shown in Figure 3D. In Figure 3D, the cerebellum in the right brain region is stimulated, enhancing coordination and balance, as shown at position 1 in Figure 3D. Additionally, the low-level visual response in the right brain region is stimulated, enhancing expressive ability, as shown at position 2 in Figure 3D.

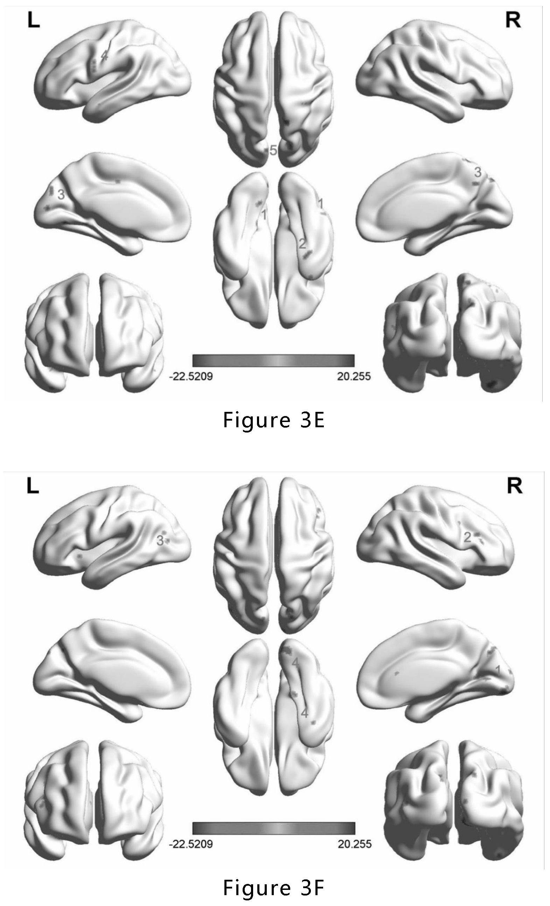

Following that, the stimulation results of the brain region after using white light with a color temperature of 4000K are shown in Figure 3E. In Figure 3E, a high-level visual response can be observed in the right brain region, enhancing the ability to convert images into meaning, as shown at position 3 in Figure 3E. Additionally, a response in the voluntary muscle control area of the right brain region can be observed, as shown at position 4 in Figure 3E. Furthermore, an enhancement in perceptual abilities, including the perception of temperature, touch, and weight, can be observed in the right brain region.

Finally, the stimulation results of the brain region after using white light with a color temperature of 5700K are shown in Figure 3F. In Figure 3F, the visual area response in the right brain region is most evident, as shown at position 2 in Figure 3F. Additionally, the language expression ability area in the right brain region shows a significant response, as shown at position 3 in Figure 3F. Furthermore, an increase in concentration and judgment abilities can be observed in the right brain region.

Based on the results of brain region stimulation under different lighting conditions, it can be observed that red light and reddish white light (3000K) elicit a response in the hippocampus of the brain; green light has a negative response in the precentral gyrus. The perceptual functions of these pure color lights corresponding to brain regions are summarized in Table 9.

**Table 9**

| Brain region | Light source | perceptual function |
|---|---|---|
| Hippocampus, Parahippocampal Gyrus | 3000K white light, pure red light | Short-term memory, Long-term memory, Spatial orientation |
| Insula Lobe | 3000~4000K white light | Empathy, Cognitive function |
| Precentral Gyrus | BOLD increase: red light, blue light | Neurons controlling the spinal cord, Somatic sensation |
| | BOLD decrease: green light | |
| Superior Frontal Gyrus | 5700K white light, blue light | Higher cognitive function, Working memory |
| Lingual Gyrus | 5700K white light | Visual signal processing, Visual memory, Analytical logic |
| IFG (right inferior frontal gyrus) | 4200K white light | Language |
| MCC (left middle cingulate cortex) | 4200K white light | Cognitive function and Reward |

This invention is based on the work of SA Rivkees published in 2003 (Developing circadian rhythmicity in infants), which indicates that infants, from birth, can have their sleep cycles effectively influenced by rhythmic lighting. This can even result in better weight gain compared to infants without rhythmic lighting. Additionally, according to AR Tarullo's 2011 work (Sleep and infant learning), the Rapid Eye Movement (REM) phase of sleep has a significant impact on the integration, development, and cognitive development of an infant's brain. A lack of REM can lead to developmental defects in the brain. Furthermore, Equivalent Melanopic Lux (EML) can be used to describe the impact of lighting on melatonin. The principle of EML calculation is to multiply the vertical visual illuminance in a space by the ratio of the light source. Therefore, EML measures the level of physiological cycle stimulation by light in a field. For example, during the day, higher EML suppresses melatonin, keeping people awake, and at night, without EML, more melatonin is secreted, aiding sleep and promoting REM. Thus, insufficient EML during the day can delay sleep cycles, reduce REM, and consequently hinder the development of the brain, nervous, and cognitive systems.

Furthermore, according to more recent research, RW Ameen's 2022 work (Early life circadian rhythm disruption in mice alters brain and behavior in adulthood) indicates that disrupting the circadian rhythm of mice through changes in lighting conditions can affect the speed of neural development in their brains.

Based on the above knowledge, this invention conducted lighting experiments with different spectra on mice, confirming that rhythmic lighting affects the sleep patterns of mice. Specifically, different light source color rendering indices (Color Rendering Index, CRI or Ra) also have varying impacts on the activity levels of mice. For example, blue light reduces the Rapid Eye Movement (REM) phase during sleep. Clearly, this invention specifically identified the spectra that promote cognitive development, as shown in Table 9. These spectra affect the brain regions corresponding to cognitive development, enhancing their BOLD responses. Additionally, from the research literature of RW Ameen and others, as well as mouse experiments, this invention proves that rhythmic lighting affects the sleep cycles of infants and newborn mammals, and from the literature, it is found that sleep cycles are related to brain and cognitive development.

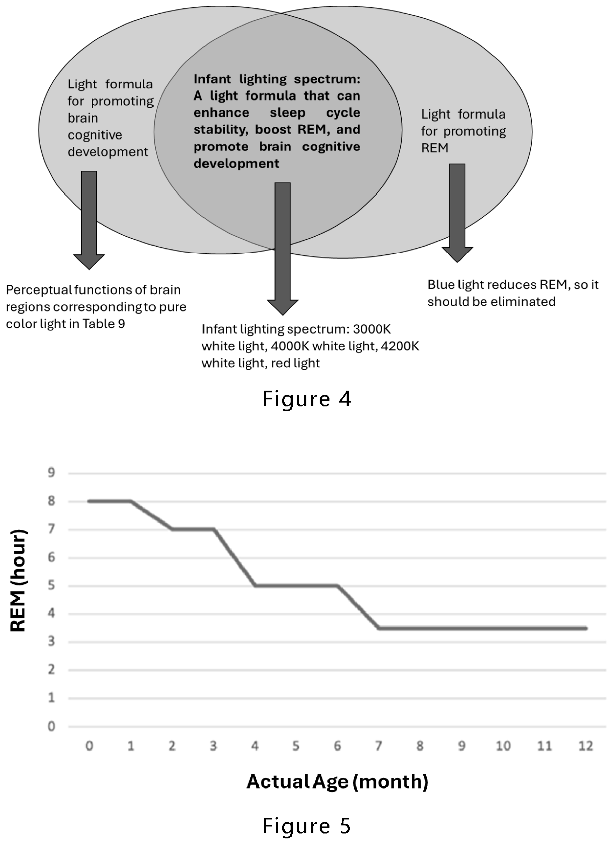

It should be particularly noted that, in order to enhance the impact of lighting on infants and toddlers in the 'spectrum that promotes brain cognitive development,' this invention further selects and screens the lighting spectrum that can 'increase EML during the day and enhance REM response at night.' Therefore, by finding the intersection of the 'spectrum that promotes brain cognitive development' and the 'lighting spectrum that increases REM response' from Table 9, this invention can obtain the spectrum formula that 'can enhance sleep cycle stability, increase REM, and promote brain cognitive development,' as shown in Figure 4. Figure 4 is a schematic diagram of the spectrum suitable for lighting for infants and toddlers. Since 'blue light reduces the REM phase during sleep,' this invention further selects from the 'spectrum that promotes brain cognitive development' in Table 9, excluding the blue light that reduces REM, to obtain the suitable lighting spectrum for infants and toddlers as shown in Table 10.

**Table 10**

| Light source | Brain region affected | Cognitive response |
|---|---|---|
| 3000K | Hippocampus, Parahippocampal Gyrus | Memory, Cognitive function |
| 4000K | Insula Lobe | Empathy, Cognitive function |
| 4200K | IFG, MCC | Language, Cognitive function and Reward |
| Red light | Precentral Gyrus, Hippocampus, Parahippocampal Gyrus | Somatic sensation, Memory, Cognitive function |

In addition, because the intensity and timing of the infant lighting spectrum must follow rhythmic lighting conditions, this invention proposes an enhanced rhythmic lighting comparison table and a regular rhythmic lighting comparison table based on the 24-hour daily light changes. The enhanced rhythmic lighting comparison table is shown in Table 11, and the regular rhythmic lighting comparison table is shown in Table 12. The rhythmic lighting comparison table mentioned here refers to the corresponding EML intensity at different times of the day. For example, the EML intensity is set to 1500 from 6 AM to 12 PM in Table 11.

**Table 11**

| Time period | EML intensity |
|---|---|
| 06:00-12:00 | 1500 |
| 12:00-18:00 | 500 |
| 18:00-22:00 | 50 |
| 22:00-06:00 | 10 |

**Table 12**

| Time period | EML intensity |
|---|---|
| 06:00-20:00 | 275 |
| 20:00-22:00 | 50 |
| 22:00-06:00 | 0 |

Since EML is the product of eye illuminance (lux) and the illuminance ratio (Scotopic/Photopic ratio, S/P ratio), i.e., EML = lux x S/P ratio; and different spectra have different S/P ratios, in other words, each light source has its corresponding S/P ratio. When the S/P ratio index is higher, it indicates better visual perception. Therefore, to enhance the infant sleep cycle and promote future cognitive development, this invention organizes the suitable lighting sources for infants and toddlers from Table 10 with the rhythmic lighting comparison tables from Table 11 and Table 12 into rhythmic lighting formulas that adjust the REM phase in the infant sleep cycle. Table 13 is the enhanced rhythmic lighting formula, and Table 14 is the regular rhythmic lighting formula. The illuminance range of +/- 10% in the tables indicates that the Lux values in Table 13 and Table 14 can have a +/- 10% error.

Using Table 13 or Table 14, this invention can adjust the rhythmic lighting formula for infants and toddlers based on their sleep cycle or REM duration standards. For example, when REM does not meet the standard, the enhanced rhythmic lighting formula can be selected to continue illuminating the infants and toddlers. For instance, when the light source is 3000K white light, it can be adjusted to the strongest EML (i.e., 1500). At this time, the system or staff can adjust the illuminance of the lamp to Lux=1071 to illuminate the infants and toddlers. When REM meets the standard, if the illumination time is in the evening, the enhanced rhythmic lighting formula with EML=50 can be selected, or if the illumination time is near midnight, the regular rhythmic lighting formula with EML=50 can be chosen. For example, when the lamp is also 3000K white light, the system or staff can adjust the illuminance of the lamp to Lux=36 to illuminate the infants and toddlers.

**Table 13**

| Light source | S/P ratio | 06:00-12:00 EML=1500 | 12:00-18:00 EML=500 | 18:00-22:00 EML=50 | 22:00-06:00 EML=10 |
|---|---|---|---|---|---|
| 3000K White light LED | 1.40 | 1071 lux | 357 lux | 36 lux | 7 lux |
| 4000K White light LED | 1.65 | 909 lux | 303 lux | 30 lux | 6 lux |
| 4200K White light LED | 1.74 | 862 lux | 287 lux | 29 lux | 6 lux |
| Red light LED | 0.50 | 3000 lux | 1000 lux | 100 lux | 20 lux |

**Table 14**

| Light source | S/P ratio | 06:00-20:00 EML=275 | 20:00-22:00 EML=50 | 22:00-06:00 EML=0 |
|---|---|---|---|---|
| 3000K White light LED | 1.40 | 179 lux | 36 lux | 0 lux |
| 4000K White light LED | 1.65 | 152 lux | 30 lux | 0 lux |
| 4200K White light LED | 1.74 | 144 lux | 29 lux | 0 lux |
| Red light LED | 0.50 | 500 lux | 100 lux | 0 lux |

Next, based on the research results from the Pathway.org institution for physical and mental development, the differentiation and explanation of REM development in newborns and infants are as follows:
1. Newborn Period (0-3 months):
   ▪ 0-1 month: At this time, about 50%-70% of a newborn's sleep is REM sleep, with an average of 8 to 12.6 hours of REM sleep per day. This period of REM sleep helps with brain integration, development, and the formation of the nervous system.
   ▪ 2-3 months: As the circadian rhythm gradually forms, the proportion of REM sleep decreases to about 40%-50%, with daily REM sleep time being around 7 to 9 hours. Sleep begins to gradually start from the NREM period, and the duration of nighttime sleep increases.
2. Infant Period (4-6 months):
   By 4 to 6 months, the proportion of REM sleep further decreases to 30%-40%, with daily REM sleep hours being about 5 to 7 hours. At this time, the infant's sleep cycle also begins to lengthen, with each sleep cycle lasting about 50 minutes.
3. Late Infant Period (7-12 months):
   ▪ By 7 to 12 months, the proportion of REM sleep decreases to levels close to those of children, accounting for about 25%-30% of total sleep time. Therefore, daily REM sleep hours are about 3.5 to 5 hours. During this stage, REM mainly occurs in the second half of the night and helps with emotional regulation and memory consolidation.

Among them, REM sleep gradually decreases with age during the first year of infants and toddlers, but it plays an important role in the integration, development, learning, and memory consolidation of the infant's brain. Therefore, based on the REM development process, this invention selects the minimum number of REM hours in each period as the minimum standard, resulting in the age and REM development curve diagram shown in Figure 5, hereinafter referred to as the "REM Development Chart." In the REM Development Chart in Figure 5, the horizontal axis represents the actual age of infants and toddlers (from birth to the 12th month), and the vertical axis represents the real-time measurement of the REM state of infants and toddlers, with REM measured in hours. For example, in the first month after birth, the infant's REM must reach at least 8 hours to contribute to the important roles of brain integration, development, learning, and memory consolidation.

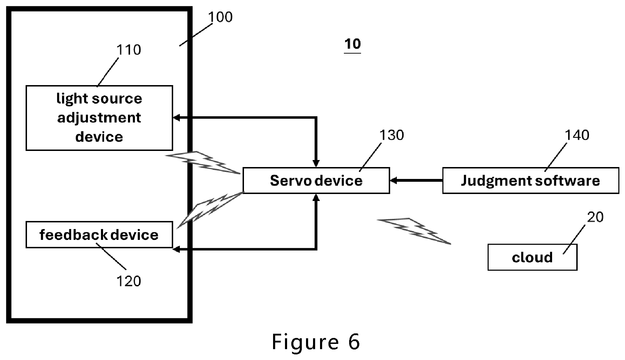

Next, this invention provides an infants and toddlers lighting adjustment system 10, as shown in Figure 6. The infant and toddlers lighting adjustment system 10 includes: an infant crib device 100, which provides a space for the infant to sleep. A light source adjustment device 110, equipped with LED lights, is arranged above or around the infant crib device 100. The light source adjustment device 110 can adjust the lighting parameters of the LED lights, including color temperature, brightness, flicker rate, and color rendering. A feedback device 120 is arranged inside or around the infant crib device 100. When the infants and toddlers lighting adjustment system 10 is in operation, the feedback device 120 is placed on the body of the infant. The feedback device 120 includes external sensors such as fMRI, EEG, BLE thermometer, GSR device, heart rate measurement device, smart mattress, and AI-equipped camera. A servo device 130 has computing, data storage, and communication functions, thus it can serve as an edge computing device. For example, an edge computing device equipped with a micro processing unit (MPU), memory, and communication module can connect to the light source adjustment device 110 and the feedback device 120 via wired or wireless communication. Additionally, judgment software 140 is configured in the micro processing unit (MPU). Based on the real-time sleep cycle and REM state of the infants and toddlers fed back by the feedback device 120, the judgment software 140 calculates the appropriate circadian lighting formula, which is then applied to the infants and toddlers through the light source adjustment device 110. The light source adjustment device 110, feedback device 120, and servo device 130 all have communication functions and can communicate with the cloud 20. In the embodiment of this invention, the cloud 20 is equipped with powerful computing and storage devices to store information on the cognitive and circadian development of infants and toddlers, suitable lighting sources for infants and toddlers in Table 10, circadian lighting comparison tables in Tables 11 and 12, circadian lighting formulas in Tables 13 and 14, and the REM development chart shown in Figure 5. Furthermore, the relevant data already stored in the storage device of the cloud 20, especially the circadian lighting formulas in Tables 13 and 14 and the REM development chart shown in Figure 5, can be transmitted to the servo device 130 for storage, making the servo device 130 an edge computing center. Moreover, the cloud 20 can perform big data AI calculations on the real-time information transmitted by the servo device 130 and then transmit the calculation results back to the servo device 130, which drives the light source adjustment device 110 to provide the appropriate circadian lighting formula for the infants and toddlers. Additionally, in another embodiment of this invention, the servo device 130 and judgment software 140 can be directly configured in the cloud. This invention is not limited to this configuration.

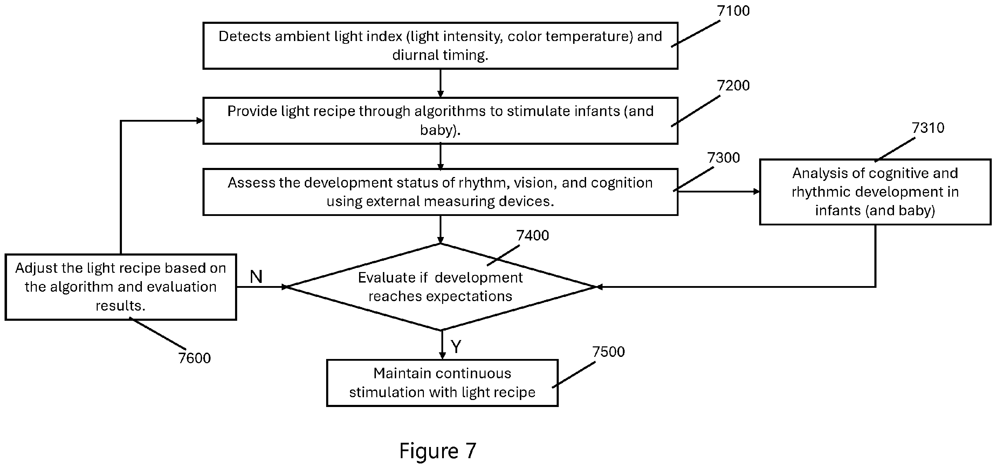

The flowchart of the operation of the infants and toddlers lighting adjustment system 10 of this invention is shown in Figure 7. The operation process of the infants and toddlers lighting adjustment system 10 includes: First, please refer to step 7100 in Figure 7. Detect the light environment index around the infant crib device 100 (e.g., the light intensity and color temperature of the background light). In the preferred embodiment of this invention, further obtain the circadian rhythm timing and detect the light environment index around the infant crib device 100 to select or adjust the appropriate circadian lighting formula for the light source adjustment device 110. The circadian rhythm information can be obtained by the servo device 130 from the cloud 20, including the suitable lighting sources for infants and toddlers in Table 10, the circadian lighting comparison tables in Tables 11 and 12, and the circadian lighting formulas in Tables 13 and 14.

Next, as shown in step 7200 of Figure 7, the infants and toddlers lighting adjustment system 10 calculates the appropriate circadian lighting formula using the algorithm configured in the judgment software 140 based on the light environment index or circadian rhythm timing. The light source adjustment device 110 then provides the circadian lighting formula from Table 13 or Table 14 to illuminate the infants and toddlers. Clearly, the adjustment of the lighting dosage considers the current light environment index. Next, the light source adjustment device 110 provides appropriate lighting to the infants and toddlers based on the circadian lighting formulas in Table 13 and Table 14, promoting a stable sleep cycle and aiding in brain integration, development, and the formation of the nervous system. In the preferred embodiment of this invention, when illuminating a newborn, the infants and toddlers lighting adjustment system 10 can choose to use different light sources from the enhanced circadian lighting formula in Table 13 sequentially. For example, the servo device 130 drives the light source adjustment device 110 to sequentially provide 3000K white light, 4000K white light, 4200K white light, and red light. During illumination, the appropriate illuminance (Lux) can be provided according to the illumination period. Additionally, the cycle of sequential illumination with different light sources can be chosen to be 1-7 days. That is, after 3000K white light provides the appropriate illuminance (Lux) to the infants and toddlers for 1-7 days according to the circadian time, it is replaced with 4000K white light to provide the appropriate illuminance (Lux) for 1-7 days, and so on. The purpose of this illumination is because infants and toddlers sleep in a space isolated from natural light and cannot perceive the circadian changes of natural light. At the same time, the sleep stages of infants and toddlers have not yet formed a circadian rhythm. Therefore, this invention provides a circadian lighting formula to help infants and toddlers have a stable sleep cycle in a fixed circadian lighting environment. With a stable sleep cycle, the brain regions of infants and toddlers can have time to rest and integrate, which is beneficial for brain development and the formation of the nervous system. Additionally, because different light sources have different cognitive responses in the brain regions, the purpose of using different light sources for cyclic circadian lighting is to stimulate the formation of more cognitive neural systems in the brain. Furthermore, since different light sources evoke varying cognitive responses in brain regions, selecting different light sources for circadian rhythm illumination aims to stimulate the formation of more cognitive neural systems in the brain. It is important to emphasize that Table 10, which lists suitable light sources for infant lighting, is merely an example embodiment of this invention. It serves to illustrate the purpose and effects of using different spectral formulas of light sources for circadian rhythm illumination and does not limit the types of light sources applicable to the circadian rhythm illumination formulas of this invention. This clarification is hereby provided.

Next, as shown in step 7300 of Figure 7, the infants and toddlers lighting adjustment system 10 evaluates the development state of the sleep cycle and cognition of infants and toddlers after the circadian lighting formula process. The evaluation method is based on the sleep state of infants and toddlers obtained from the external sensors in the feedback device 120. For example, EEG (electroencephalogram) can directly observe the REM sleep state and define sleep quality. The BLE thermometer uses core body temperature as a standard to judge sleep cycle and REM duration, thus determining sleep quality. The GSR device uses emotional stability time as a standard to judge sleep cycle and REM duration, thus determining sleep quality. Additionally, feedback from the smart mattress and AI-equipped camera is used to confirm and record the REM sleep state of infants and toddlers under circadian lighting. In a preferred embodiment, this invention uses an IoT (Internet of Things) communication architecture to evaluate and statistically monitor the REM sleep state of infants and toddlers in real-time or periodically.

It should be noted that in the preferred embodiment of this invention, the invention has already evaluated and statistically monitored the REM sleep state curve of infants and toddlers based on the feedback data from the feedback device 120, particularly using fMRI or EEG, through real-time and periodic monitoring. The AI-equipped camera records the REM sleep state of each infant and toddler after illumination. Subsequently, the servo device 130 stores the REM sleep state curve of each infant and toddler after illumination in the database of the cloud 20. In the preferred embodiment of this invention, this REM sleep state curve of infants and toddlers can be refined with an increasing number of samples and through the AI learning model, establishing results or curves that better align with the cognitive and circadian development analysis of infants and toddlers, as shown in step 7310 of Figure 7. Additionally, step 7310 can provide the REM development curve of infants and toddlers shown in Figure 5, serving as a standard comparison curve to determine whether the REM sleep state of each infant and toddler after illumination meets the expected hours. This REM development curve shown in Figure 5 can be stored in the memory of the servo device 130 or the cloud 20.

In the analysis of the REM sleep state curve mentioned above, this invention specifically targets infants and toddlers, including newborns. The goal is to help newborns establish a more resilient REM sleep state through the periodic stimulation of early circadian lighting formulas, given that newborns have not yet formed a circadian rhythm. This can help correct circadian rhythm disturbances caused by staying up late or occasional jet lag more quickly as infants and toddlers grow. It also helps the nervous system of infants and toddlers to develop a certain level of resilience.

Next, as shown in step 7400 of Figure 7, the infants and toddlers lighting adjustment system 10 needs to determine whether the REM sleep state curve of the infants and toddlers after the illumination process conforms to the REM sleep development curve (i.e., the REM sleep standard curve). The standard curve of the REM sleep state can be provided by step 7310.

In step 7400, when a newborn less than one month old undergoes the illumination process with 3000K white light, the feedback device 120 records the current REM sleep state of the newborn as 10 hours. At this time, the servo device 130 retrieves the standard REM development curve for infants and toddlers from the memory (as shown in step 7310). The micro processing unit (MPU) then extracts the standard REM sleep state curve, indicating that the REM sleep requirement for infants and toddlers less than one month old should be more than 8 hours. Therefore, when the REM sleep state of this newborn is 10 hours, the servo device 130 determines that it meets the expected standard, and the light source adjustment device 110 continues the illumination process as per the procedure, as shown in step 7500. Meanwhile, the servo device 130 also transmits the current REM sleep state information (10 hours) to the servo device 130 for recording, as shown in step 7310.

Continuing in step 7400, when a newborn less than one month old undergoes the illumination process with 3000K white light, the feedback device 120 records the current REM sleep state as 6 hours. At this time, the micro processing unit (MPU) in the servo device 130 determines that the current REM sleep state does not meet the expected standard. Therefore, the algorithm in the servo device 130 provides appropriate illumination to the infants and toddlers based on the circadian lighting formulas in Table 13 and Table 14, as shown in step 7600. For example, if the REM sleep state does not meet the standard of 6 hours, the illumination can be adjusted to a higher illuminance circadian lighting formula from Table 13 to continue illuminating the infants and toddlers. For instance, when the light source is 3000K white light, it can be adjusted to the highest EML (i.e., 1500). At this time, the system or staff can adjust the illuminance of the light source adjustment device 110 to Lux=1071 to illuminate the infants and toddlers. When the REM sleep state meets the standard (i.e., REM reaches more than 8 hours), if the illumination time is in the evening, EML=50 from Table 13 can be chosen, or if the illumination time is near midnight, EML=50 from the regular circadian lighting formula in Table 14 can be chosen. For example, when the light source adjustment device 110 provides 3000K white light, the system or staff can adjust the illuminance to Lux=36 to illuminate the infants and toddlers. Additionally, if the newborn is already being illuminated with 4200K white light and the MPU determines that the current REM sleep state does not meet the expected standard, the algorithm in the servo device 130 can choose the spectral formula with EML=1500 or EML=500 for illumination. For example, when EML=500 is chosen, the illuminance of the light source adjustment device 110 is adjusted to Lux=357 to illuminate the infants and toddlers. Clearly, the illumination process of this invention uses periodic stimulation with circadian lighting formulas to help newborns establish a more resilient REM sleep state.

Next, in step 7600, if a newborn has sequentially completed the illumination process with the circadian lighting formulas from Table 13 and has reached the infant period (4-6 months) or late infant period (7-12 months), this invention can use the algorithm in the servo device 130 to select specific light sources that achieve perceptual effects for illuminating the infant. For example, the algorithm may choose to use 4200K white light to enhance the infant's language and cognitive functions. Alternatively, the servo device 130 can select the light source based on the parents' choices or requests. For instance, if the parents choose to enhance the infant's language function, the algorithm will select 4200K white light for illumination. Subsequently, when the infant reaches one year old, the REM development curve for each infant can be obtained in step 7310. Clearly, although each infant's REM development curve is based on the standard REM development curve in Figure 5, the REM development curve for each infant and toddler is not entirely identical. When combined with information from specific light source illumination, this invention can provide a learning curve for perceptual functions during the infant and toddler period.

Since newborns or infants have not yet formed a circadian rhythm, their sleep time is randomly distributed between day and night, accompanied by irregular sleep and wake patterns. Therefore, according to the spectral formula with circadian lighting provided by this invention, the sleep cycle of infants and toddlers can be influenced through illumination. The spectral formula for illumination is adjusted based on changes in the REM phase of the sleep cycle of infants and toddlers to enhance their sleep cycle and promote future cognitive development.

Furthermore, for infants and toddlers who do not yet have cognitive functions, providing illumination with color temperature cycle adjustment can improve and enhance their sleep quality. Good sleep quality can help establish a robust biological rhythm through early periodic lighting stimulation, particularly by stimulating the development of the intrinsically photosensitive retinal ganglion cells (ipRGCs) in infants and toddlers. For example, low-level visual stimulation can enhance expressive abilities, while high-level visual stimulation can improve the ability to convert images into meaningful concepts.

Clearly, this invention targets infants and toddlers whose intrinsically photosensitive retinal ganglion cells (ipRGCs) have not yet established a cycle influenced by actual lighting conditions. Through early periodic stimulation, it can help newborns establish a more resilient circadian cycle. This allows for quicker correction of circadian rhythm disturbances caused by staying up late or occasional jet lag as infants and toddlers grow. It also helps the nervous system of infants and toddlers develop a certain level of resilience.

Finally, it should be emphasized once again that the above description is only the preferred embodiment of this invention and is not intended to limit the scope of the invention's claims. Additionally, the above description should be understood and implemented by those with ordinary knowledge in the relevant technical field. Therefore, any equivalent changes or modifications completed without departing from the disclosed concepts of this invention should be included within the scope of the patent claims of this invention.

## Claims

1. A lighting method for infant including:
providing a lighting adjustment system, consists of a light source adjustment device, a feedback device, and a servo device, the servo device stores a rhythmic lighting formula related to perceptual development and a REM sleep development curve, used to drive the light source adjustment device to provide lighting according to the rhythmic lighting formula, the feedback device comes into contact with the infant or toddler to transmit the number of REM sleep hours, after light therapy, to the servo device;
executing the illumination procedure, driven by the servo device, which adjusts the light source according to the rhythmic lighting formula to sequentially illuminate the infant;
obtaining the number of REM sleep hours of the infant or toddler, transmitted by the feedback device to the servo device after illumination;
evaluating whether the REM sleep state meets expectations, by comparing the number of REM sleep hours transmitted by the feedback device with the REM sleep development curve stored in the servo device, and
maintaining continuous illumination according to the rhythmic lighting formula, the servo device determines that the number of REM sleep hours of the infant or toddler meets the REM sleep development curve, it drives the light source adjustment device to continue illuminating the infant or toddler according to the rhythmic lighting formula.

2. The lighting method according to claim 1, wherein the rhythmic lighting formula is as shown in the table below.
| Light source | S/P ratio | 06:00-12:00 EML=1500 | 2:00-18:00 EML=500 | 18:00-22:00 EML=50 | 22:00-06:00 EML=10 |
|---|---|---|---|---|---|
| 3000K white light LED | 1.40 | 1071 lux | 357 lux | 36 lux | 7 lux |
| 4000K white light LED | 1.65 | 909 lux | 303 lux | 30 lux | 6 lux |
| 4200K white lightLED | 1.74 | 862 lux | 287 lux | 29 lux | 6 lux |
| red light LED | 0.50 | 3000 lux | 1000 lux | 100 lux | 20 lux |

3. The lighting method according to claim 1, wherein the rhythmic lighting formula is as shown in the table below.
| Light source | S/P ratio | 06:00-20:00 EML=275 | 20:00-22:00 EML=50 | 22:00-06:00 EML=0 |
|---|---|---|---|---|
| 3000K white light LED | 1.40 | 179 lux | 36 lux | 0 lux |
| 4000K white light LED | 1.65 | 152 lux | 30 lux | 0 lux |
| 4200K white light LED | 1.74 | 144 lux | 29 lux | 0 lux |
| red light LED | 0.50 | 500 lux | 100 lux | 0 lux |

4. The lighting method according to claim 1, wherein the hours on the REM sleep development curve decrease as the actual age of the infant or toddler increases.

5. The lighting method according to claim 1, wherein before further executing the illumination according to the rhythmic lighting formula, the servo device detects the light environment index and circadian rhythm information around the infant or toddler.

6. The lighting method according to claim 1, wherein the servo device determines that the number of REM sleep hours of the infant or toddler is lower than the hours on the REM sleep development curve, the servo device selects the rhythmic lighting formula with high equivalent melatonin illuminance (EML) from the light source to execute the illumination.

7. The lighting method according to claim 1, wherein the feedback device is selected from a combination of EEG, BLE thermometer, and GSR devices.

8. The lighting method according to claim 1, wherein the comparison of the infant or toddler 's REM sleep hours with the REM sleep development curve hours is performed by an algorithm configured in the servo device.

9. A lighting adjustment system for infant, comprising:
a crib device providing a sleeping space for the infant;
a light source adjustment device equipped with LED lights, positioned above or around the crib device, for adjusting the lighting parameters of the LED lights;
a feedback device placed on the body of the infant or toddler to obtain and transmit the infant's real-time REM sleep state; and
a servo device equipped with a processing unit, a communication unit, and a memory unit, where the memory unit stores rhythmic lighting formulas related to perceptual development and a REM sleep development curves, the processing unit is used to drive the light source adjustment device to illuminate the infant or toddler based on the rhythmic lighting formula table.

10. The lighting adjustment system according to claim 9, wherein the processing unit in the servo device is further configured with an algorithm, the servo device receives the real-time REM sleep status of the infant or young toddler from the feedback device, the algorithm compares the number of REM sleep hours of the infant or toddler after illumination with the hours on the REM sleep development curve.
